# EUROPEAN PATENT APPLICATION

(11) **EP 1 685 850 A1**
(43) Date of publication of application: **02.08.2006**
(21) Application number: 05001997.5
(22) Date of filing: 01.02.2005
(51) Int. Cl.: A61K 51/04, A61K 51/12, C12Q 1/04, G01N 33/62

(54) **A method for the diagnosis of helicobacter pylori infection and diagnostic kit for performing the method**

(71) Applicant: Aygen, Sitke, Dr., 51105 Köln (DE)
(72) Inventor: Aygen, Sitke, Dr., 51105 Köln (DE)
(74) Representative: Werner, Hans-Karsten

(57) **Abstract**

The method for the diagnosis of *Helicobacter pylori* infection by the oral administration of defined amounts of ¹³C-labeled urea together with a sufficient amount of an acid bringing down the pH of the solution and thereafter examining the ¹³C content in the urea breast test (¹³C-UBT), is improved

in that patients taking proton-pumping-inhibitors (PPIs) are administered higher amounts of acids,
overcompensating for a time from 10 minutes to one hour the activity of the PPI.

## Description

The present invention relates to a method for the diagnosis of *Helicobacter pylori* infection by the oral administration of defined amounts of ¹³C-labeled urea together with a sufficient amount of an acid bringing down the pH of the solution and thereafter examining the ¹³C-content in the urea breast test (¹³C-UBT) and a diagnostic kit for performing the method.

This method was compared with other known methods as the upper digestive tract endoscopy, the stool antigen test (HpSA /Meridian, Milan, Italy), serological method (Pylortest EIA-G III / Orion Diagnostics, Espoo, Finland), antibody detection in urine (Otsuka Diagnostic, Frankfurt, Germany) with the result, that the Helicobacter Test INFAI (INFAI GmbH, Cologne, Germany) became the leading method. It is in commercial use with 75mg ¹³C-labeled urea together with up to 1g citric acid for adults and with 45mg ¹³C-labeled urea together with 100ml orange juice for children from the age of 3 years.

A severe disadvantage of nearly all these tests, however, is that proton pump inhibitors (PPIs) and antacid therapy disturb and give false negative results; see L. Gatta et al, in American Journal of Gastroenterology, 2004 , P.823 -828.

It therefore is demanded, that these PPIs and antacid therapy must be discontinued 12 to 14 days at least before the ¹³C-UBT can be performed with reliable results. This increases cost and inconvenience, as the patient must return after at least 12 days. Furtheron the patient suffers from the discontinuation during this time.

It has been the object of the invention to provide a method for the diagnosis of *Helicobacter pylori* for patients taking PPIs or other antacid drugs without discontinuation of the therapy.

Surprisingly it now was found, that this disadvantage can be overcome if the patients taking proton pumping-inhibitors (PPIs) are administered higher amounts of acids overcompensating for a time of 10 minutes to one hour the activity of the PPI.

It could not be foreseen that such a short-time overcompensation of the PPI-activity would be able to overcome the known long-time negative influence of these drugs on the reliability of the UBT-tests.

There are strong indications that this is also helpful with patients being administered antacid drugs.

It is not only more convenient for the patients to be tested immediately without discontinuation of intake of PPIs or antacid drugs. It is also better accepted to suffer for at most one hour instead of two weeks without the therapy.

The amount of acid necessary for overcompensation of PPIs was found in the range of 4 to 8 g of an pharmacologically acceptable acid. Such acids are for example citric acid, maleic acid, acetic acid, and phosphoric acid. 5 to 6 g citric acid were found to be fully sufficient.

These acids are administered preferably in form of aqueous solutions.

Such solutions can be prepared from solid or liquid acids immediately before the test. They also can be prepared in larger amounts and can be stored.

A preferred way of administration is also the preparation of an aqueous solution of the acid already containing the ¹³C-urea.

The breath test is run in the usual and well established method described in the literature and the patient instruction sheets, which are available also to the medical doctor. The breath samples are analysed for example by gas isotope ratio mass spectroscopy or infrared spectrometer.

The administration of these higher amounts of acids for patients taking PPIs or antacid drugs may also helpful for the test examining the ¹³C-content in serum as described for example in WO 03/14744.

The following examples compare the results of the test according to the present invention with 5 g citric acid (New INFAI ¹³C UBT) with the tests according to the prior art (INFAI ¹³C -UBT Standard with 1g citric acid). 152 consecutive Helicobacter pylori positive patients have been studied.

Upper endoscopy was performed in each patient. During endoscopy, multiple biopsies were taken from antrum and corpus. Histology, RUT (Rapid Urease Test), and culture were used to assess the *Helicobacter pylori* status, Histology was performed using the H&E, and the modified Giemsa stain.

Histology was scored using the Up date Sydney System. Patients were considered infected if 2 out of 3 tests were positive or if culture was positive alone.

Patients found to be positive were randomised using a randomisation list to perform a standard ¹³C-UBT or the new ¹³C-UBT formulation.

After UBT they received a standard dose of esomeprazole (40 mg/day) to be taken in the morning 30 min before breakfast.

Patients were asked to return at day 14^{th} and 28^{th} of treatment with esomeprazole to perform the UBTs and to check the compliance with medication.

Patients were also asked to return at day 7^{th} and 14^{th} after stopping treatment with esomeprazole to perform the UBTs.

Sensitivity, difference between proportions for independent samples and their Cis (Confidence intervals) were calculated according to Wilson and Newcombe method's.

Sensitivity was assessed according to ITT (Intension to Treat) Analysis i.e. patients dropped were considered as False Negative results.

The p value was calculated using Fischer's exact test. NNT was also calculated. Multiple logistic regression for the independent determinants of sensitivity was performed using the following variables:
Type of UBT
Sex
Age
Body Mass Index
Score for *Helicabacter pylori* in Antrum (density)
Score for *Helicobacter pylori* in Corpus (density)

Multiple logistic regression was performed according to ITT Analysis i.e. patients dropped were considered as False Negative results.

The results after 14^{th} day of PPI

From 152 *Helicobacter pylori* positive patients randomised 76 patients were tested with the new formulation and 76 patients were tested with the formulation of the prior art.

The test according to the invention gave 70 True Positive, 3 False Negative , 2 Drop-Out.

The standard gave 53 True Positive, 17 False Negative , and 3 Drop-Out.

The sensitivity increased from 75,7% to 95,8% with a p value of 0.0005.

The results after 28^{th} day of PPI were
66 True Positive, 5 False Negative, 2 Drop-Out with the new formulation and
37 True Positive, 32 False Negative, 3 Drop-Out.

New Test: 2 new False Negative and standard Test, 15 new False Negative. The sensitivity increased from 53,6% to 92,9% with a p value of 0.0001

The results after 7^{th} day of wash-out were
New Test: 69 True Positive, 2 Dropout and standard Test: 67 True Positive 3 Drop-Out

The results after 14^{th} day of wash-out were
New Test: 67 True Positive, 2 Drop-Out and standard Test: 67 True Positive, 3 Drop-Out.

These results clearly show the highly significant improvement with the new formulation and confirm the known demand of at least 12 day discontinuation of PPI treatment. This now can be avoided with the new formulation according to the present invention.

## Claims

1. A method for the diagnosis of *Helicobacter pylori* infection by the oral administration of defined amounts of ¹³C-labeled urea together with a sufficient amount of an acid bringing down the pH of the solution and thereafter examining the ¹³C content in the urea breast test (¹³C-UBT), **characterized in that** patients taking proton-pumping-inhibitors (PPIs) are administered higher amounts of acids,
overcompensating for a time from 10 minutes to one hour the activity of the PPI.

2. The method according to claim 1, **characterized in that** the patients are administered 4 to 8 g of a pharmacologically acceptable acid.

3. The method according to claim 1 or 2, **characterized in that** the patients are administered an aqueous solution of an acid selected from the group of citric acid, maleic acid, tartaric acid, acetic acid, and phosphoric acid.

4. The method according to one of the claims 1 to 3, **characterized in that** the patients are administered an aqueous solution of 5 to 6 g of citric acid.

5. A diagnostic kit for performing the method according to any of claims 1 to 4, consisting of
a) either an acidic aqueous solution containing exactly from 10 to 100 mg of ¹³C-urea and 4 to 8 g of a pharmacologically acceptable acid, or
b) an urea container with exactly from 10 to 100 mg of ¹³C-urea and a pack of 4 to 8 g of a solid or liquid acid,
c) a patient instruction sheet.
